# EUROPEAN PATENT APPLICATION

(11) **EP 4 521 418 A1**
(43) Date of publication of application: **12.03.2025**
(21) Application number: 23861653.6
(22) Date of filing: 18.07.2023
(51) Int. Cl.: G16H 50/30, G16H 20/00, G16H 50/20

(54) **SYSTEM AND METHOD FOR PROVIDING HEALTH PROMOTION PROGRAM**

(71) Applicant: IIDA Group Holdings Co., Ltd., Musashino-shi, Tokyo 180-0013 (JP)
(72) Inventor: MORI Kazuhiko, Musashino-shi Tokyo 1800013 (JP); MATSUMOTO Koichi, Musashino-shi Tokyo 1800013 (JP)
(74) Representative: Mewburn Ellis LLP
(86) International application number: PCT/JP2023/026231
(87) International publication number: WO 2025/017824

(57) **Abstract**

To provide a system and method that can effectively utilize the biometric data obtained in the daily life of users for maintaining and promoting the health of users. The system includes a first sensor installed in a first facility that continuously collects biometric data of the user, a second sensor installed in a second facility that continuously collects the biometric data which is the same kind of data as the first sensor from the user, an analysis device that composes to perform analysis including comparison of the biometric data collected by the first sensor and the second sensor, and to determine the health condition of the user, and a program generation device that composes to generate a health promotion program according to the user based on the determination result of the health condition.

## Description

### TECHNICAL FIELD

The present invention relates to a system and method for providing a health promotion program according to the health condition of users including residents of a house.

### BACKGRAUND ART

In recent years, a smart house has been attracting attention. The smart house generally refers to a house that uses information technology (IT) to control appliances that use electricity and gas, such as lighting fixtures, cooking appliances, and air conditioning equipment, thereby to optimally control energy consumption.

For example, Patent Document 1 (Japanese Unexamined Patent Publication No. 2013-15271) discloses an indoor environment control system for controlling an indoor environment according to the comfort of each person in a room without enlarging time and energy cost even in a simple system, which includes an environment information detection part, a part for detecting information on a person in the room, a control part, and an operation part. In the indoor environment control system, a storing means of the control part includes a previously prepared database for storing the relationship of information on the person in the room and the comfort of the person in the room. A calculating means of the control part generates a regulation signal for controlling a fluctuation of the operation part by regarding the information of the person in the room as a fluctuation index so that the comfort of the person in the room is optimum in the database on the basis of the information of the person in the room detected with the part for detecting the information of the person in the room.

Further, Patent Document 2 (Japanese Patent No. 6539799) discloses a safety confirmation system for accurately determining abnormality of a resident and respond more safely, more reliably, and more swiftly when the abnormality occurs. The safety confirmation system includes a first sensor that is installed in a residence and detects at least a resident's heart beat and respiratory rate without contact; an abnormality determination unit that detects significant difference in heart rate and respiratory rate from normal; a communication unit that performs safety confirmation by communicating between an operator and the resident; an arrival information presentation unit that presents the operator with arrival of an ambulance crew to the residence due to a dispatch request by the operator; and a remote unlocking unit that urgently unlocks the key of the residence according to instructions of the operator.

### PRIOR ART REFERENCE

### Patent Reference

Patent Document 1: Japanese Unexamined Patent Publication No. 2013-15271
Patent Document 2: Japanese Patent No. 6539799

### SUMMARY OF THE INVENTION

### Problem to be solved by the invention

The indoor environment control system of Patent Document 1 is limited to environmental control. The safety confirmation system of Patent Document 2 is limited to confirmation of safety. These technologies are only a limited use of collected biometric data. Furthermore, these technologies utilize a wearable sensor to collect the biometric data, but there is a person who feels hassle of the putting on and taking off the wearable sensor, and such an operation complicated. Therefore, problems as to usability remain in these technologies.

Therefore, the object of the present invention is to provide a system and method that can effectively utilize the biometric data obtained in the daily life of users including residents of a house for maintaining and promoting the health of users.

### MEANS TO SOLVE THE PROBLEMS

In order to solve the above-mentioned problems, one embodiment of the present invention provides a system for providing a health promotion program for a user, which is characterized by including:
a first sensor installed in a first facility that continuously collects biometric data of the user,
a second sensor installed in a second facility that continuously collects biometric data which is the same kind of data as the first sensor from the user,
an analysis device that composes to perform analysis including comparison of the biometric data collected by the first sensor and the second sensor, and to determine the health condition of the user, and
a program generation device that composes to generate the health promotion program according to the user based on the determination result of the health condition.

Here, the biometric data includes dynamic data. Further, the health promotion program includes, other than exercise programs, various programs and various health information for maintaining and promoting the physical and mental health of the user.

In the system of the present invention, it is preferable that the biometric data is at least one kind of data which show facial expressions, heart rate, oxygen saturation, carbon dioxide exhaled amount, surface body temperature, core body temperature, skin protein analysis, body composition, autonomic nerves, HbA1c, internal water content, foot pressure distribution, walking posture, walking speed, change in joint range of motion, fluctuation in center of gravity, amount of activity, myoelectricity, electrocardiography, brain waves, standing and sitting posture, body temperature, blood pressure, blood flow, heart rate, breathing, sweating, eyeballs, sleeping time, amount and time of excretion, blood components, urine components, saliva components, intraoral images, and fecal components.

Further, in the system of the present invention, it is preferable that
the analysis device performs the procedures where
the human biometric data which includes at least one kind of data, which data shows the human facial expressions, heart rate, oxygen saturation, carbon dioxide exhaled amount, surface body temperature, core body temperature, skin protein analysis, body composition, autonomic nerves, HbA1c, internal water content, foot pressure distribution, walking posture, walking speed, change in joint range of motion, fluctuation in center of gravity, amount of activity, myoelectricity, electrocardiography, brain waves, standing and sitting posture, body temperature, blood pressure, blood flow, heart rate, breathing, sweating, eyeballs, sleeping time, amount and time of excretion, blood components, urine components, saliva components, intraoral images, and fecal components, and the human health condition including the energy consumption and exercise effect of the person are used as a teaching data, to generate a predictive model where the input is the human biometric data, and the output is the health condition of the person by using a machine learning, and
the health condition of the user is output from the collected biometric data by using the predictive model.

Further, in the system of the present invention, it is preferable that
the program generation unit performs the procedures where
the biological characteristics of the user are extracted by comparing the collected biometric data with a predetermined evaluation index, and
the predetermined evaluation index and the evaluation values indicating the effectiveness of the health promotion program are used as a teaching data, to generate an evaluation model where the input is the biometric data, and the output is the evaluation to the health promotion program by using a machine learning, and
a health promotion program presented to the user is output from the collected biometric data by using the evaluation model together with biological characteristics of the user.

Further, the other embodiment of the present invention provides a method characterized in that,
in the method for providing a health promotion program for a user, a processer and a computer perform the procedures of:
continuously collecting biometric data of the user in a first sensor installed in a first facility,
continuously collecting the biometric data which is the same kind of data as the first sensor from the user in a second sensor installed in a second facility,
performing analysis including comparison of the biometric data collected by the first sensor and the second sensor, and determining the health condition of the user, and
generating a health promotion program according to the user based on the results of the determination of the health condition.

### EFFECT OF THE INVENTION

According to the present invention, it is possible to effectively utilize the biometric data obtained in the daily life of users for maintaining and promoting the health of the users.

### BRIEF EXPLANATION OF DRWAINGS

FIG. 1 is a schematic diagram showing the overall image of an example of the present embodiment.
FIG. 2 is a block diagram showing the overall configuration of the system 1 which provides the health promotion program.
FIG. 3 is a flowchart showing the processing procedures which provide the health promotion program.

### MODE FOR CARRYING OUT THE INVENTION

In the following, by referring the drawings, the typical embodiments of the system and method according to the present invention are explained in detail. However, the present invention is not particularly limited to the embodiments shown in the drawings. Further, since these drawings are presented to explain the concept of the present invention, there are cases where ratios and numbers are exaggerated or simplified as necessary for ease of understanding.

### 1. Overall image of the present embodiment

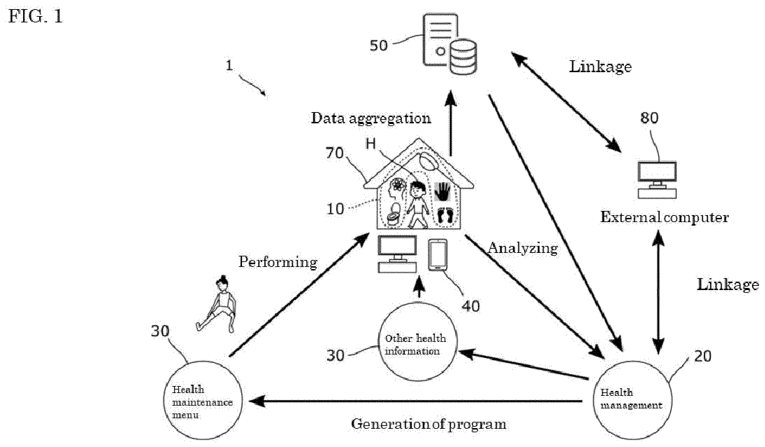

The overall image surrounding the system according to the present embodiment will be described with reference to FIG. 1.

The facility in the present embodiment (the present invention) is a concept that includes any facility (also referred to as facility, institution, site, place, or activity base, etc.) in which the system according to the present invention can be utilized. Examples of facilities include residences, workplaces, educational facilities such as schools, medical facilities such as hospitals and health checkup centers, nursing facilities, care homes, nursing homes, residential facilities for the elderly, exercise facilities, recreational facilities, gathering places, and accommodation facilities, but are not limited thereto. The biometric data of the users who spend their daily lives in these facilities is sensed by the sensors placed at various locations in these facilities. For example, for the users engaged in transportation services, the workplace may include mobile objects such as vehicles, ships, and aircraft.

Here, the biometric data refers to all data that can be obtained from the body of the user. The biometric data includes not only dynamic data such as walking posture, walking speed, change in joint range of motion, fluctuation in center of gravity and amount of activity, but also time-series data such as myoelectricity, electrocardiography and brain waves, static data such as standing and sitting posture, body temperature, blood pressure and blood flow, heart rate, breathing, sweating, eyeballs, sleeping time, amount and time of excretion, blood components, urine components such as blood sugar level, saliva components, intraoral (tongue, pharynx, etc.) images, and fecal components. Further, the residence refers to a building in which the person lives on a daily basis, and includes not only single-family houses and apartment complexes, but also so-called old people's homes.

The health condition of the user is then determined by computer analysis of the obtained biometric data. For example, by comparing the same kind of biometric data, that is, the first and second biometric data, sensed in different facilities, that is, the first and second facilities, it is possible to exactly evaluate the health conditions of the users under the different environments.

To give a specific example, when the measured values of blood pressure, heart rate, respiratory rate, and perspiration at the workplace of the user are greater than the corresponding values at the residence of the user by the set values, it is possible to evaluate that the user is under excessive strain at the workplace.

Alternatively, when the measured values of blood pressure, heart rate, respiratory rate, blood flow, and perspiration at the exercise facility are greater than the corresponding values at the residence of the user by the predetermined acceptable degree, it is possible to evaluate that the user performs moderate exercise.

Alternatively, when the measured or estimated values of distance traveled, speed of travel, and metabolism at the workplace of the user are smaller than or equal to the corresponding values at the residence of the user, it is possible to evaluate that the user is in the state of insufficient exercise at the workplace.

Alternatively, when the measured values of blood pressure, heart rate, and respiratory rate at the accommodation and recreational facility are smaller than the corresponding values at the residence of the user by more than the set value, it is possible to evaluate that the user is in a reasonably relaxed state at the accommodation and recreational facility.

Alternatively, when the measured values of the brain wave in the educational facility or workplace of the user are different from the corresponding value in the residence of the user, the user may be evaluated as being in a state of lack of concentration.

Next, the health promotion program customized for each user is generated based on the determination result of the health condition. Here, the generated health promotion program includes, other than exercise programs, various programs and various health information for maintaining and promoting the physical and mental health of the user.

To give a specific example, for the user who is evaluated as being under excessive strain at the workplace, efforts to alleviate the strain, such as improving the work, taking holidays, exercising moderately, getting enough sleep, and consulting a medical doctor, are suggested. In this case, an exercise menu may be presented according to the health condition of the user.

Alternatively, for the user who is evaluated to be in the state of insufficient exercise at the workplace, for example, the moderate exercise is suggested.

This health promotion program is presented to the user, and the program is performed by the user.

By repeating the above-described sensing, determination of health condition, generation of the health promotion program, and performance by the user, the health function of the user is promoted.

In addition, the collected biometric data may be aggregated in a data server or the like, presented to the medical personnel such as medical doctors with the consent of the user, and utilized by the medical personnel. Further, the generated determination result of the health condition may be presented to the medical personnel and utilized by the medical personnel with the consent of the user.

Furthermore, the collected biometric data and the determination result of the generated health condition may be utilized by government and local communities, with the consent of the users, for creating a more comfortable and livable community, for managing the health of the users, and for enabling the users to continue living independently in their own homes in old age.

The system 1 according to the present embodiment includes function units which realize a sensing function that collects the biometric data from the user in the facility, an analysis and determination function that determines the health condition of the user by analyzing the collected biometric data, and a program generation function that generates the health promotion program based on the determination result of the health condition.

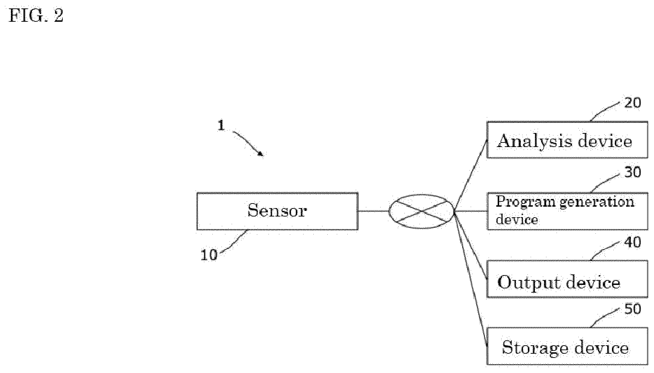

For realizing such functions, the system 1 includes a sensor 10, an analysis device 20, a program generation device 30, an output device 40, and a storage device 50 (see FIG. 2). Each of these components is communicatively connected via a wired or wireless communication network. The sensor 10, the analysis device 20, the program generation device 30, the output device 40, and the storage device 50 may be separate units or may be configured integrally. Further, the storage device 50 may be installed as a component of a data center (not shown), and the data center may store not only the collected data but also the collected samples (for example, saliva and fecal samples). In this case, the data center can also be called a biobank.

These components will be explained in detail below.

### 2. Sensor

The sensor 10 is a general term for various sensors that are installed in the facility 70 such as a residence and continuously collect biometric data of the user H. The sensor 10 transmits the collected biometric data to the storage device 50 together with the collection date and time, and the data stores. The storage device 50 may be a data server, but is not limited thereto. Note that the sensor 10 may transmit and store the collected biometric data to a storage (not shown) in the facility 70.

Examples of usable sensors 10 include infrared temperature cameras, hyperspectral imaging cameras, RFID sensors, pressure sensors, motion capture devices, temperature sensors, component analyzers, weight sensors, flow rate sensors, and position sensors (all not shown), and the like, but are not limited thereto.

More specifically, the infrared temperature camera is installed, for example, in rooms such as a living room, study, bedroom, and working room of the facility 70, and can measure the facial expressions, heart rate, oxygen saturation, carbon dioxide exhaled amount, surface body temperature, and core body temperature of the user H.
The hyperspectral imaging camera is installed, for example, at an entrance and the vicinity thereof, and, other than the personal authentication function of the user H, can analyze, with respect to the user H, the skin protein analysis, body composition (for example, including composition ratio of fat / bone / lean soft tissue, etc., and body weight, body fat, basal metabolism, visceral fat, muscle mass, bone mass) and autonomic nerves (for example, including autonomic nerve fatigue level, sympathetic and parasympathetic nerve balance). Alternatively, the hyperspectral imaging camera is installed on a dining table or in a kitchen, and the vicinity thereof, and can measure glycohemoglobin (HbA1c). The RFID sensor is installed on an indoor wall, and can measure the internal water content of the user H. The pressure sensor is installed, for example, on a hallway floor or an indoor floor, and can measure the foot pressure distribution of the user H. The motion capture device is installed, for example, in an indoor hallway, and can measure the walking posture, walking speed, changes in joint range of motion, changes in the center of gravity, amount of activity of the user H.

The sensing of the biometric data of the user H by the sensor 10 is performed, for example, by constant measurement.

It is preferable that the sensor 10 is installed in a hidden manner so that the user H cannot immediately discover. This is to prevent the user H from becoming nervous upon noticing the presence of the sensor 10 and from causing the sensed biometric data to deviate from the daily values of the user H. In other words, the purpose is to know the user H in a natural state in daily life.

Other than the sensor 10, a sensor for obtaining the environmental data may be installed. That is, a thermometer to measure the room temperature, a hygrometer to measure the humidity in the room, an illuminance meter to measure the brightness of the room, a carbon dioxide meter to measure the CO₂ concentration in the room, etc. may be installed. The environmental data collected by these sensors is transmitted and stored in the storage device 50. Note that the sensor 10 may include these sensing functions.

Additionally, the biometric data of the user may be obtained by utilizing various sensors built into wearable devices and portable information terminals such as smartphones. This type of biometric data is useful as an aid to determine the health condition of the user. The sensor 10 may include these sensing functions.

The collected biometric data, the environmental data and the other data are transmitted to and stored in the storage device 50. The storage device 50 may store the determination result of the health condition and the generated health promotion program, which will be described later. Various data stored in the storage device 50 may be made available to an external computer 80 used by a doctor and a medical institution in charge of the user H under the permission of the user H. Through such medical cooperation, various health records necessary for diagnosis and treatment of the user H can be shared among the medical personnel in a timely manner. Of course, the data linkage may also include medical doctors, dentists, pharmacists, or dietitians, and is not limited to the medical personnel.

### 3. Analyzing Device

The analysis device 20 determines the health condition of the user H by analyzing the biometric data collected by the sensor 10.

The analysis device 20 can suitably utilize an AI analysis for the data analysis.

That is, the analysis device 20 uses the human biometric data which includes at least one kind of data, which data shows the human facial expressions, heart rate, oxygen saturation, carbon dioxide exhaled amount, surface body temperature, core body temperature, skin protein analysis, body composition, autonomic nerves, HbA1c, internal water content, foot pressure distribution, walking posture, walking speed, change in joint range of motion, fluctuation in center of gravity, amount of activity, myoelectricity, electrocardiography, brain waves, standing and sitting posture, body temperature, blood pressure, blood flow, heart rate, breathing, sweating, eyeballs, sleeping time, amount and time of excretion, blood components, urine components, saliva components, intraoral images, and fecal components, and the human health condition including the energy consumption and exercise effect of the person as a teaching data, and generate a predictive model where the input is the human biometric data, and the output is the health condition of the person by using a machine learning. And the analysis device 20 outputs the health condition of the user from the collected biometric data by using the predictive model.

For example, the analysis device 20 can use at least one kind of data among the biometric data such as human facial expressions, heart rate, oxygen saturation, carbon dioxide exhaled amount, surface body temperature, core body temperature, skin protein analysis, body composition, autonomic nerves, HbA1c, internal water content, foot pressure distribution, walking posture, walking speed, changes in joint range of motion, fluctuations in center of gravity, and amount of activity, as a teaching variable (teaching data) as an existing index. That is, the analysis device 20 can conduct a machine learning by using the biometric data of the same type as the data group collected by the sensor 10 as explanatory variables to generate an analysis program. Then, the analysis device 20 can algorithmically analyze the biometric information such as facial expressions, heart rate, oxygen saturation, carbon dioxide exhaled amount, surface body temperature, core body temperature, skin protein analysis, body composition, autonomic nerves, HbA1c, internal water content, foot pressure distribution, and the motor function information such as joint angle during walking and the time-related change thereof, lower limb muscle strength, center of gravity, or three dimensional moving distance of the observation point correlated thereto, walking posture, walking speed, and amount of activity, by using the basic information such as age, gender, height and weight, as parameters to be utilized.

The analysis device 20 obtains the objective evaluation including the health function and motor function as the health condition of the user H, by applying the sensing data to the above-mentioned model. An example of the machine learning that can be utilized in the present embodiment is a deep learning, but it is not limited thereto.

The analysis result 20 transmits the determination result of the health condition of the user H to the storage device 50, and then stores therein. Needless to say, the determination result can be effectively used by the medical personnel with the permission of the user H. Further, by outputting the determination result of the health condition of the user H to the output device 40 described later, the user H and the family thereof can detect illness or poor physical condition of the user H at an early stage, and can utilize for health management of the user H. That is, the determination result can be effectively utilized to support the user H and to link with related parties.

### 4. Program Generation Device

The program generation device 30 generates the health promotion program customized for each user H based on the determination result of the health condition obtained by the analysis device 20.

That is, the program generation device 30 extracts the biological characteristics of the user by comparing the collected biometric data with the predetermined evaluation index. Then, the program generation device 30 uses the predetermined evaluation index and the evaluation values indicating the effectiveness of the health promotion program are used as the teaching data, and generates the evaluation model where the input is the biometric data, and the output is the evaluation to the health promotion program by using teh machine learning. Then, the program generation device 30 outputs the health promotion program which is presented to the user from the collected biometric data by using the evaluation model together with biological characteristics of the user.

Here, the health promotion programs are roughly classified into those related to health functions and those related to motor functions.

For example, regarding the health functions, the program generation device 30 clarifies the biological characteristics of the user H by comparing the normal range for each individual with evaluation indicators such as epidemiology and medical guidelines, with respect to the facial expressions, heart rate, oxygen saturation, carbon dioxide exhaled amount, surface body temperature, core body temperature, skin protein analysis, body composition, autonomic nerves, HbA1c, internal water content, and foot pressure distribution. Then, the program generation device 30 can construct the algorithm related to each individual lifestyle habits by performing the AI analysis on the measured data, the basic information such as age, gender, height and weight, and the living environment data (for example, biometric data collected by the sensor 10) of the user H, and can present the behavioral changes and the effects associated therewith.

Further, regarding the motor functions, the program generation device 30 clarifies the characteristics of the dynamic analysis of the user H by comparing the positions and velocities of each joint, joint angles and angular velocities. Then the program generation device 30 can construct the motion measurement algorithms, based on the measured data, in addition to estimated energy consumption and exercise effects during exercise, and can present the recommended exercises, and the energy consumption and exercise effects during exercise.

An example of the AI analysis that can be used here is the deep learning, but it is not limited thereto.

The program generation device 30 transmits the generated health promotion program to the storage device 50 and stores therein. Needless to say, such a program can be shared among medical personnel with the approval of the user H. Further, by outputting the program to the output device 40, the user H and the family thereof can clearly understand what they should do to maintain, recover, and improve the health functions and motor functions of the user H, and can utilize for the health management of the user H.

Note that the analysis device 20 and the program generation device 30 can be configured as one or more computers including an arithmetic circuit such as central processing unit (CPU), as well as a random-access memory (RAM) and a read-only memory (ROM). The functions of the analysis device 20 and the program generation device 30 described above can be realized by reading an execution program stored in the ROM into the RAM and executing by the CPU. The analysis device 20 and the program generation device 30 determine the health condition and generate the health promotion program periodically or at a timing deemed necessary for each individual, by using continuously collected biometric data.

The output device 40 is a device that outputs the determination result of the health condition and the health promotion program. The output device 40 includes the terminal (including smartphone, personal computer), display, printer, projector, speaker of the user H. For example, when the determination result of the health condition indicates an abnormality in the health condition of the user H, that is, a deviation from the normal range (for example, with respect to the data expressed in numerical values, when the result is above or below the predetermined threshold, or when being out of range), the output device 40 may output an alert to the terminal of the user H and the related parties. Note that the output device 40 may display other information related to daily life, such as information regarding energy and housing, and regional information.

### 5. Operation of Health Promotion Program Providing System

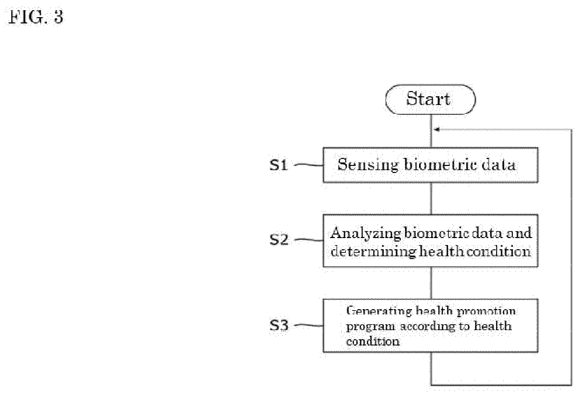

The processing procedures for providing the health promotion program by the system 1 will be explained with reference to FIG. 3.

Firstly, in step S1, the sensor 10 senses the biometric data of the user H and stores in the storage device 50 in association with the sensing time. Sensing is performed continuously at predetermined time intervals.

Then, in step S2, the analysis device 20 analyzes the collected biometric data, determines the health condition of the user H, and stores the determination result in the storage device 50. For example, the analysis device 20 generates the learned model in advance by the machine learning, and obtains the health condition of the user H by applying the biometric data of the user H to the learned model. Step S2 is performed periodically or at a timing deemed necessary for each individual.

When the determination result of the health condition is generated in the analysis device 20, in step S3, the program generation device 30 generates the health promotion program for each user H based on the determination result of the health condition, and stores the program in the storage device 50.

The determination result of the health condition and the health promotion program are displayed on the output device 40. Thereby, the user H can understand own health condition (for example, health functions and motor functions) and can clearly understand what to do to maintain, recover, and improve own health functions and motor functions. The user H can practice the presented health promotion program and utilize for own health management.

Then, return to sensing and repeat the above procedures. Thereby, the user H can effectively utilize the biometric data obtained in daily life to promote the health functions of the user H. Furthermore, the user H can understand the effects of performance of the health promotion program through the determination result of subsequent health condition.

The thus accumulated sensing data, the determination result of the health condition, and the health promotion program are stored in the storage device 50 and shared among medical personnel involved with the user H with the approval of the user H. Thereby, it is expected that this will allow the medical doctors to perform diagnosis and treatment more appropriately and quickly. Such effects can be expected not only during face-to-face medical treatment but also in remote medical treatment, and more fulfilling medical linkage will be realized.

Further, by sharing the health information of the user H mentioned above among those involved in care, it is expected that more appropriate and prompt care support will be provided.

Of course, it is also possible to use the sensing data and the determination result of the health condition as a monitoring system for the user H or an abnormality detection system.

Further, by utilizing the collected biometric data and the determination result of the health condition by the government and the local community with consent of the user H, it is possible to reflected in policies to create a more comfortable and livable community.

Since the sensor 10 is installed in the facility 70, there is no need to worry about attachment and detachment unlike wearable sensors. Furthermore, since the sensor 10 is hidden from the eyes of the user H, the user H is not aware of the existence of the sensor 10. Therefore, the daily biometric data of the user H can be obtained, and the health condition of the user H can be understood with high reliability.

In the above, although the typical embodiments of the present invention have been described, the present invention is not limited to these, and various design changes are possible, and all of those are included in the present invention.

### INDUSTRIAL APPLICABILITY

According to the present invention, it is possible to effectively utilize the biometric data obtained in the daily life of users for maintaining and promoting the health of the users. The utilization of the biometric data includes support for the users, and information linkage with the medical and care staffs.

### Explanation of Symbols

- 1: Health promotion program providing system
- 10: Sensor
- 20: Analysis device
- 30: Program generation device
- 40: Output device
- 50: Storage device
- H: User

## Claims

1. A system for providing a health promotion program for a user, which is **characterized by** including:
a first sensor installed in a first facility that continuously collects biometric data of the user,
a second sensor installed in a second facility that continuously collects biometric data which is the same kind of data as the first sensor from the user,
an analysis device that composes to perform analysis including comparison of the biometric data collected by the first sensor and the second sensor, and to determine the health condition of the user, and
a program generation device that composes to generate the health promotion program according to the user based on the determination result of the health condition.

2. The system according to claim 1, wherein the biometric data is at least one kind of data which show facial expressions, heart rate, oxygen saturation, carbon dioxide exhaled amount, surface body temperature, core body temperature, skin protein analysis, body composition, autonomic nerves, HbA1c, internal water content, foot pressure distribution, walking posture, walking speed, change in joint range of motion, fluctuation in center of gravity, amount of activity, myoelectricity, electrocardiography, brain waves, standing and sitting posture, body temperature, blood pressure, blood flow, heart rate, breathing, sweating, eyeballs, sleeping time, amount and time of excretion, blood components, urine components, saliva components, intraoral images, and fecal components.

3. The system according to claim 2, wherein the analysis device performs the procedures where
the human biometric data which includes at least one kind of data, which data shows the human facial expressions, heart rate, oxygen saturation, carbon dioxide exhaled amount, surface body temperature, core body temperature, skin protein analysis, body composition, autonomic nerves, HbA1c, internal water content, foot pressure distribution, walking posture, walking speed, change in joint range of motion, fluctuation in center of gravity, amount of activity, myoelectricity, electrocardiography, brain waves, standing and sitting posture, body temperature, blood pressure, blood flow, heart rate, breathing, sweating, eyeballs, sleeping time, amount and time of excretion, blood components, urine components, saliva components, intraoral images, and fecal components, and the human health condition including the energy consumption and exercise effect of the person are used as a teaching data, to generate a predictive model where the input is the human biometric data, and the output is the health condition of the person by using a machine learning, and
the health condition of the user is output from the collected biometric data by using the predictive model.

4. The system according to claim 3, wherein the program generation unit performs the procedures where
the biological characteristics of the user are extracted by comparing the collected biometric data with a predetermined evaluation index, and
the predetermined evaluation index and the evaluation values indicating the effectiveness of the health promotion program are used as a teaching data, to generate an evaluation model where the input is the biometric data, and the output is the evaluation to the health promotion program by using a machine learning, and
a health promotion program presented to the user is output from the collected biometric data by using the evaluation model together with biological characteristics of the user.

5. A method **characterized in that**,
in the method for providing a health promotion program for a user, a processer and a computer perform the procedures of:
continuously collecting biometric data of the user in a first sensor installed in a first facility,
continuously collecting the biometric data which is the same kind of data as the first sensor from the user in a second sensor installed in a second facility,
performing analysis including comparison of the biometric data collected by the first sensor and the second sensor, and determining the health condition of the user, and
generating a health promotion program according to the user based on the results of the determination of the health condition.
